# EUROPÄISCHE PATENTSCHRIFT

(11) **EP 0 729 453 B1**
(45) Veröffentlichungstag und Bekanntmachung des Hinweises auf die Patenterteilung: **06.03.2002**
(21) Anmeldenummer: 95900739.4
(22) Anmeldetag: 15.11.1994
(51) Int. Cl.: C07D 201/08

(54) **VERFAHREN ZUR HERSTELLUNG VON CAPROLACTAM**
PROCESS FOR MANUFACTURING CAPROLACTAM
PROCEDE DE PREPARATION DE CAPROLACTAME

(30) Priorität: 20.11.1993 DE 4339648
(43) Veröffentlichungstag der Anmeldung: 04.09.1996
(73) Patentinhaber: BASF AKTIENGESELLSCHAFT, 67056 Ludwigshafen (DE)
(72) Erfinder: FUCHS, Eberhard, D-67227 Frankenthal (DE); WITZEL, Tom, D-67069 Ludwigshafen (DE)
(86) Internationale Anmeldenummer: EP9403781
(87) Internationale Veröffentlichungsnummer: WO9514664

(56) Entgegenhaltungen:
- EP-A- 0 150 295
- FR-A- 2 029 540
- US-A- 2 301 964
- US-A- 4 625 023
- US-A- 4 628 085

## Beschreibung

Die vorliegende Erfindung betrifft ein neues Verfahren zur Herstellung von cyclischen Lactamen durch Umsetzung von Aminocarbonsäurenitrilen mit Wasser in Gegenwart von Katalysatoren.

Aus der US-A 4 628 085 ist die Umsetzung von 6-Aminocapronsäurenitril mit Wasser in der Gasphase an saurem Kieselgel bei 300°C bekannt. Als Produkt der quantitativ verlaufenden Umsetzung wird mit einer anfänglichen Selektivität von 95 % Caprolactam erhalten, doch ist ein schneller Produktivitäts- und Selektivitätsrückgang festzustellen. Ein ähnliches Verfahren wird in der US-A 4 625 023 beschrieben, nach der ein hochverdünnter Gasstrom aus 6-Aminocapronsäurenitril, Adipinsäuredinitril, Ammoniak, Wasser und Trägergas über ein Kieselgel- und ein Kupfer/Chrom/ Barium-Titanoxid Katalysatorbett geleitet wird. Bei 85 % Umsatz wird Caprolactam mit einer Selektivität von 91 % erhalten. Auch hier ist eine schnelle Katalysatordesaktivierung zu beobachten.

Gegenstand der US-A 2 301 964 ist die nicht katalysierte Umsetzung von 6-Aminocapronsäurenitril zu Caprolactam in wäßriger Lösung bei 285°C. Die Ausbeuten liegen unter 80 %.

Die FR-A 2 029 540 beschreibt ein Verfahren zur Cyclisierung von 6-Aminocapronsäurenitril zu Caprolactam mittels homogener Metallkatalysatoren aus der Zink- und Kupfergruppe in wäßriger Lösung, wobei Caprolactam in Ausbeuten bis zu 83 % erhalten wird. Die vollständige Abtrennung des Katalysators vom Wertprodukt Caprolactam bereitet jedoch Probleme, da dieses Komplexe mit den verwendeten Metallen bildet.

Aufgabe der vorliegenden Erfindung war es daher, ein Verfahren zur Herstellung cyclischer Lactame durch Umsetzung von Aminocarbonsäurenitrilen mit Wasser zur Verfügung zu stellen, das die vorstehend beschriebenen Nachteile nicht mit sich bringt.

Diese Aufgabe wird erfindungsgemäß dadurch gelöst, daß die Umsetzung in flüssiger Phase in Gegenwart heterogener Katalysatoren auf der Basis von Titandioxid, Zirkonoxid, Ceroxid und Aluminiumoxid durchgeführt wird.

Bevorzugte Ausführungsformen des erfindungsgemäßen Verfahrens sind den Unteransprüchen zu entnehmen.

Als Ausgangsstoffe im erfindungsgemäßen Verfahren werden Aminocarbonsäurenitrile, vorzugsweise solche der allgemeinen Formel I 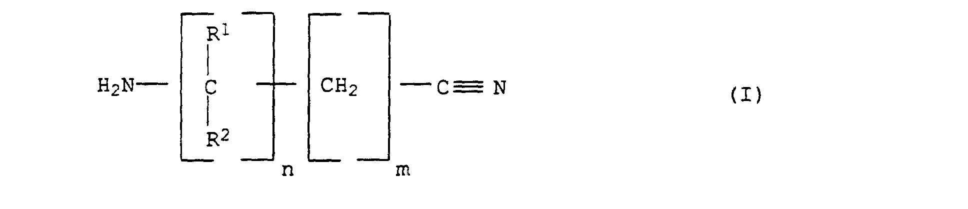 eingesetzt, wobei n und m jeweils die Werte 0, 1, 2, 3, 4, 5, 6, 7, 8 und 9 haben können und die Summe aus n + m mindestens 3, vorzugsweise mindestens 4 beträgt.

R¹ und R² können prinzipiell Substituenten jeglicher Art sein, wobei lediglich sichergestellt sein sollte, daß die gewünschte Cyclisierungsreaktion durch die Substituenten nicht beeinflußt wird. Vorzugsweise sind R¹ und R² unabhängig voneinander C₁-C₆-Alkyl- oder C₅-C₇-Cycloalkylgruppen oder C₆-C₁₂-Arylgruppen.

Besonders bevorzugte Ausgangsverbindungen sind Aminocarbonsäurenitrile der allgemeinen Formel

H₂N―(CH₂)ₘ―C≡N

wobei m einen Wert von 3, 4, 5 oder 6, insbesondere 5 aufweist.
Für m = 5 ergibt sich als Ausgangsverbindung 6-Aminocapronsäurenitril.

Nach dem erfindungsgemäßen Verfahren werden die vorstehend beschriebenen Aminocarbonsäurenitrile mit Wasser in flüssiger Phase unter Verwendung heterogener Katalysatoren zu cyclischen Lactamen umgesetzt. Bei Verwendung von Aminocarbonsäurenitrilen der Formel I erhält man die entsprechenden cyclischen Lactame der Formel II 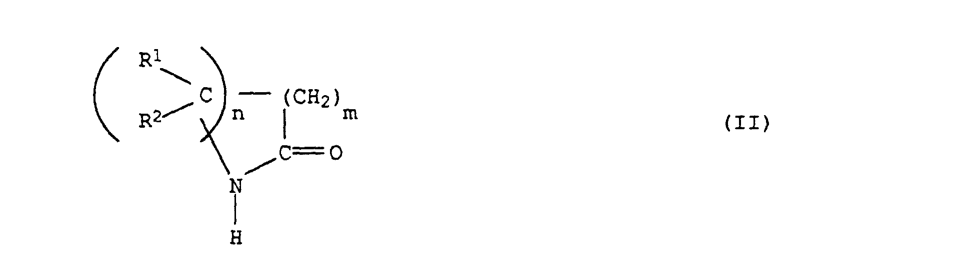 wobei n, m, R¹ und R² die vorstehend genannte Bedeutung haben. Besonders bevorzugte Lactame sind solche, in denen n = O ist und m einen Wert von 4,5 oder 6 hat, insbesondere 5 (im letzteren Fall erhält man Caprolactam).

Die Umsetzung wird in flüssiger Phase bei Temperaturen von im allgemeinen 140 bis 320°C, vorzugsweise 160 bis 280°C, durchgeführt; der Druck liegt im allgemeinen im Bereich von 1 bis 250 bar, vorzugsweise von 5 bis 150 bar, wobei darauf zu achten ist, daß das Reaktionsgemisch unter den angewandten Bedingungen zum überwiegenden Teil flüssig ist. Die Verweilzeiten liegen im allgemeinen im Bereich von 1 bis 120, vorzugsweise 1 bis 90 und insbesondere 1 bis 60 min. In einigen Fällen haben sich Verweilzeiten von 1 bis 10 min als völlig ausreichend erwiesen.

Pro mol Aminocarbonsäurenitril werden im allgemeinen mindestens 0,01 mol, vorzugsweise 0,1 bis 20 und insbesondere 1 bis 5 mol Wasser eingesetzt.

Vorteilhaft wird das Aminocarbonsäurenitril in Form einer 1 bis 50 gew.-%igen, insbesondere 5 bis 50 gew.-%igen, besonders vorzugsweise 5 bis 30 gew.-%igen Lösung in Wasser (wobei dann das Lösungsmittel gleichzeitig Reaktionspartner ist) oder in Wasser/ Lösungsmittel-Gemischen eingesetzt. Als Lösungsmittel seien beispielhaft Alkanole wie Methanol, Ethanol, n- und i-Propanol, n-, i- und t-Butanol und Polyole wie Diethylenglykol und Tetraethylenglykol, Kohlenwasserstoffe wie Petrolether, Benzol, Toluol, Xylol, Lactame wie Pyrrolidon oder Caprolactam oder alkylsubstituierte Lactame wie N-Methylpyrrolidon, N-Methylcaprolactam oder N-Ethylcaprolactam sowie Carbonsäureester, vorzugsweise von Carbonsäuren mit 1 bis 8 C-Atomen genannt. Auch Ammoniak kann bei der Reaktion anwesend sein. Selbstverständlich können auch Mischungen organischer Lösungsmittel Anwendung finden. Mischungen aus Wasser und Alkanolen im Gewichtsverhältnis Wasser/Alkanol 1-75/25-99, vorzugsweise 1-50/50-99 haben sich in einigen Fällen als besonders vorteilhaft herausgestellt.

Als Katalysatoren, die unter den oben beschriebenen Reaktionsbedingungen sehr hohe Umsätze, Ausbeuten, Selektivitäten und Standzeiten besitzen, sind heterogene Katalysatoren auf Basis Titanoxid, Zirkonoxid, Ceroxid und Aluminiumoxid zu nennen. Diese können in Form von Pulvern, Gries, Splitt, Strängen oder zu Tabletten gepreßt, verwendet werden. Die Form der Oxide richtet sich in der Regel nach den Erfordernissen der jeweiligen Reaktionsführung, wobei in Suspension Pulver oder Gries verwendet wird. Bei der Festbettfahrweise werden üblicherweise Tabletten oder Stränge mit Durchmessern zwischen 1 mm und 10 mm verwendet.

Aluminiumoxid ist in allen Modifikationen, die durch Erhitzen der Vorläuferverbindungen Aluminiumhydroxid (Gibbsit, Böhmit, Pseudo-Böhmit, Bayerit und Diaspor) bei unterschiedlichen Temperaturen erhalten werden können, geeignet. Dazu gehören insbesondere gamma- und alpha-Aluminiumoxid und deren Gemische.

Die Oxide können in reiner Form (Gehalt des jeweiligen Oxids > 80 Gew.-%), als Gemisch der oben genannten Oxide, wobei die Summe der oben genannten Oxide > 80 Gew.-% betragen soll, oder als Trägerkatalysator, wobei die oben genannten Oxide auf einen mechanisch und chemisch stabilen Träger meist mit hoher Oberfläche aufgebracht werden können, verwendet werden.

Die reinen Oxide können durch Fällung aus wäßrigen Lösungen hergestellt worden sein, z.B. Titandioxid nach dem Sulfatprozeß oder durch andere Verfahren wie die pyrogene Herstellung von feinen Aluminiumoxid-, Titandioxid- oder Zirkondioxid-Pulvern, die käuflich zu erhalten sind.

Zur Herstellung von Gemischen der verschiedenen Oxide stehen mehrere Methoden zur Wahl. Die Oxide oder deren Vorläuferverbindungen, die durch Calzinieren in die Oxide umwandelbar sind, können z.B. durch eine gemeinsame Fällung aus Lösung hergestellt werden. Dabei wird im allgemeinen eine sehr gute Verteilung der beiden verwendeten Oxide erhalten. Die Oxid- oder Vorläufergemische können auch durch eine Fällung des einen Oxids oder Vorläufers in Gegenwart des als Suspension von fein verteilten Teilchen vorliegenden zweiten Oxids oder Vorläufers ausgefällt werden. Eine weitere Methode besteht im mechanischen Mischen der Oxid- oder Vorläuferpulver, wobei dieses Gemisch als Ausgangsmaterial zur Herstellung von Strängen oder Tabletten Verwendung finden kann.

Zur Herstellung von Trägerkatalysatoren bieten sich verschiedene Methoden an. So können die Oxide in Form ihrer Sole durch einfaches Tränken auf dem Träger aufgebracht werden. Durch Trocknen und Calzinieren werden die flüchtigen Bestandteile des Sols üblicherweise aus dem Katalysator entfernt. Solche Sole sind für Titandioxid, Aluminiumoxid und Zirkondioxid käuflich erhältlich.

Eine weitere Möglichkeit zum Aufbringen von Schichten der aktiven Oxide besteht in der Hydrolyse oder Pyrolyse von organischen oder anorganischen Verbindungen. So kann ein keramischer Träger mit Titandioxid durch Hydrolyse von Titan-Isopropylat oder anderen Ti-Alkoxiden in dünner Schicht belegt werden. Weitere geeignete Verbindungen sind unter anderen TiCl4, Zirkonylchlorid, Aluminiumnitrat und Cernitrat. Geeignete Träger sind Pulver, Stränge oder Tabletten der genannten Oxide selbst oder anderer stabiler Oxide wie Siliciumdioxid. Die verwendeten Träger können zur Verbesserung des Stofftransports makroporös ausgestaltet sein.

Nach dem erfindungsgemäßen Verfahren erhält man cyclische Lactame, insbesondere Caprolactam in hoher Ausbeute bei guten Selektivitäten und guter Konstanz der Katalysatoraktivität.

### Beispiele

### Beispiele 1 bis 6

In einen geheizten Rohrreaktor von 25 ml Inhalt (Durchmesser 6 mm; Länge 800 mm), der mit Titandioxid (Anatas) in Form von 1,5 mm Strängen gefüllt war, wurde bei 100 bar eine Lösung von 6-Aminocapronsäurenitril (ACN) in Wasser und Ethanol in den in der Tabelle angegebenen Gewichtsverhältnissen geleitet. Der den Reaktor verlassende Produktstrom wurde gaschromatographisch und hochdruckflüssigchromatographisch (HPLC) analysiert. Die Ergebnisse sind ebenfalls der Tabelle zu entnehmen.

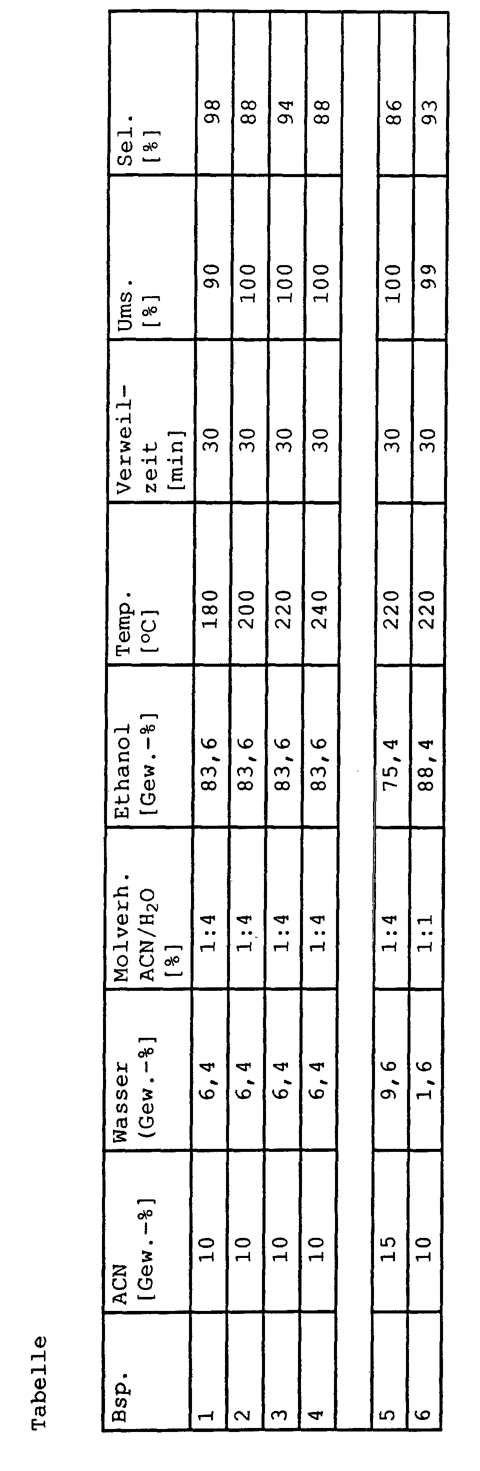

### Vergleichsversuch

Entsprechend den in Beispiel 1 beschriebenen Versuchen wurde eine Lösung von 10 % Aminocapronsäurenitril. 6,4 % Wasser und 83,6 % Ethanol ohne heterogenen Katalysator bei 250°C und einer Verweilzeit von 30 min in einem leeren Rohrreaktor umgesetzt. Der Umsatz betrug 28 % und die Selektivität zu Caprolactam 74 %.

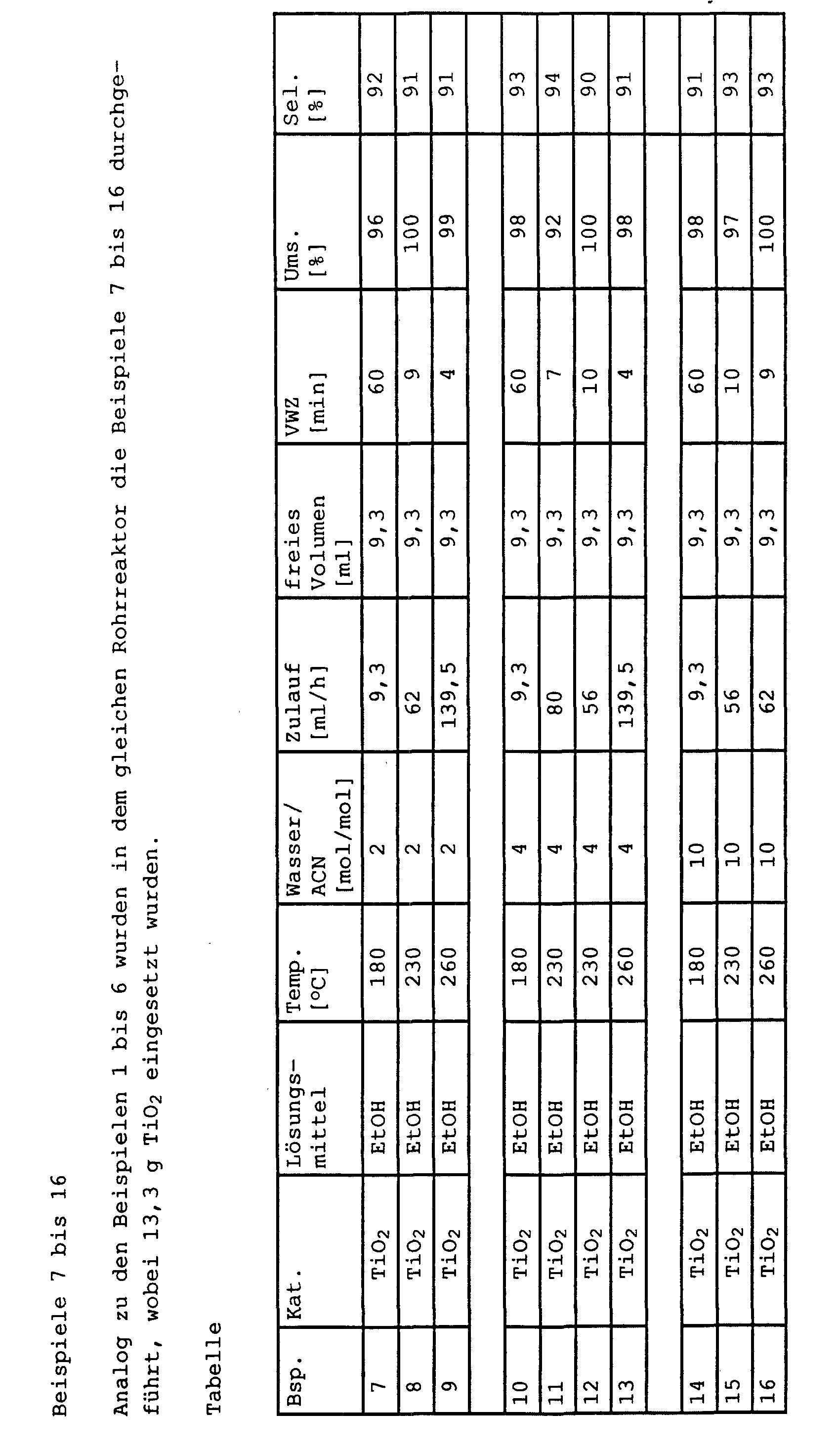

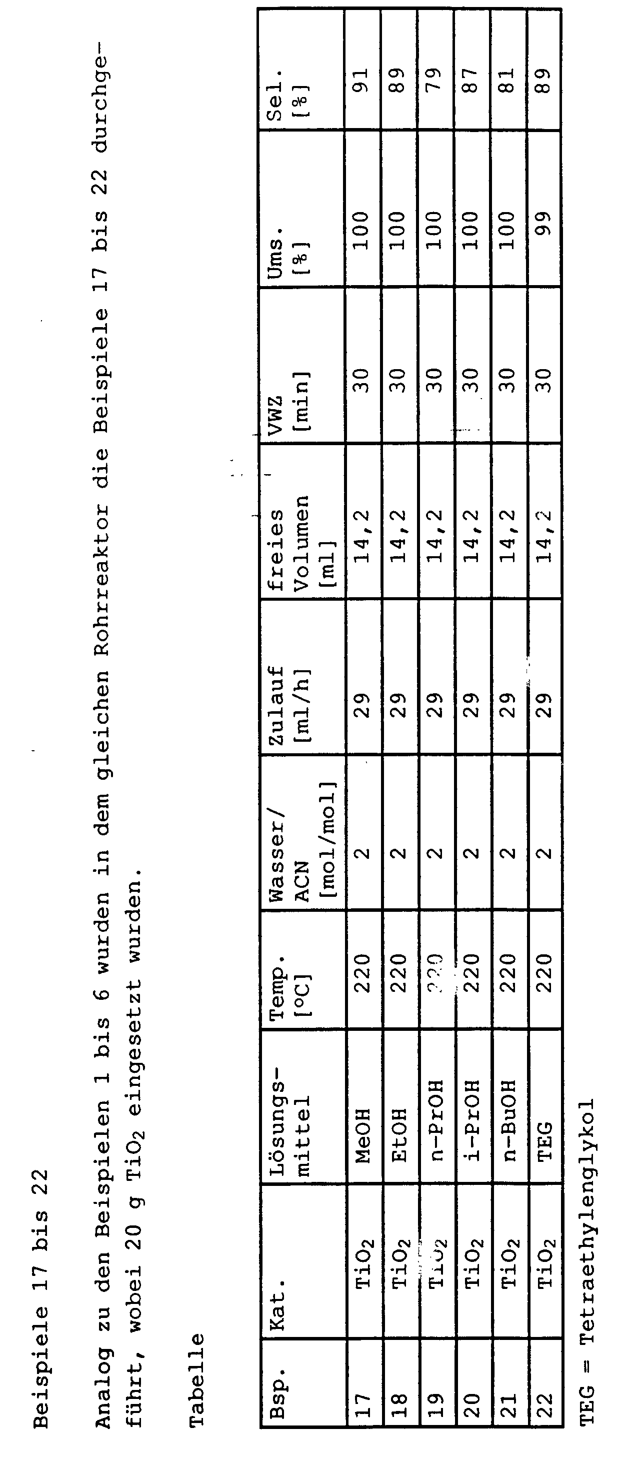

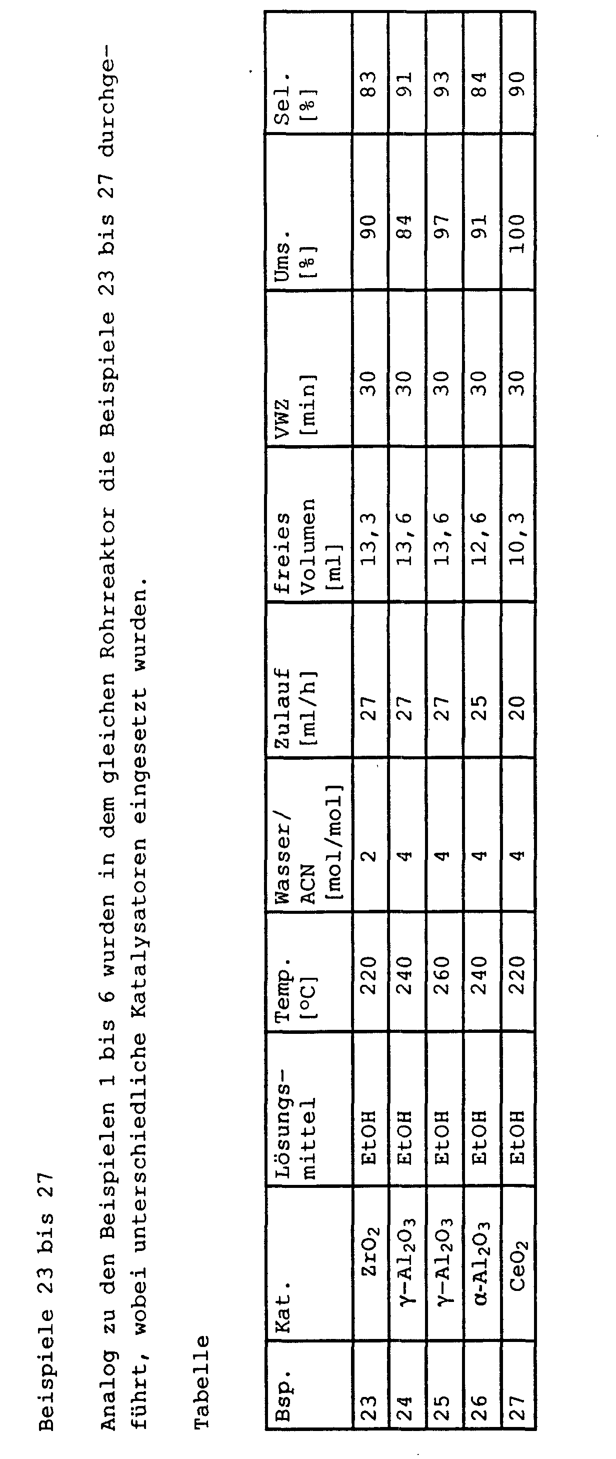

## Patentansprüche

1. Verfahren zur Herstellung cyclischer Lactame durch Umsetzung von Aminocarbonsäurenitrilen mit Wasser in Gegenwart von Katalysatoren, **dadurch gekennzeichnet, daß** man die Umsetzung in flüssiger Phase in Gegenwart heterogener Katalysatoren auf der Basis von Titandioxid, Zirkonoxid, Ceroxid und Aluminiumoxid durchführt.

2. Verfahren nach Anspruch 1, **dadurch gekennzeichnet, daß** man die Umsetzung bei einer Temperatur im Bereich von 140 bis 320°C durchführt.

3. Verfahren nach einem der Ansprüche 1 bis 2, **dadurch gekennzeichnet, daß** man Aminocarbonsäurenitrile der Formel
H₂N―(CH₂)ₘ―C≡N
wobei
m 3, 4, 5 oder 6 ist, einsetzt.

4. Verfahren nach Anspruch 3, **dadurch gekennzeichnet, daß** man als Aminocarbonsäurenitril 6-Aminocapronsäurenitril einsetzt.

5. Verfahren nach einem der Ansprüche 1 bis 4, **dadurch gekennzeichnet, daß** man eine 1 bis 50 gew.-%ige Lösung des Aminocarbonsäurenitrils in Wasser oder in Wasser/org. Lösungsmittel-Gemischen einsetzt.

## Claims

1. A process for preparing cyclic lactams by reacting amino carbonitriles with water in the presence of catalysts, wherein the reaction is carried out in liquid phase in the presence of heterogeneous catalysts based on titanium dioxide, zirconium oxide, cerium oxide and aluminum oxide.

2. A process as claimed in claim 1, wherein the reaction is carried out at from 140 to 320°C.

3. A process as claimed in either of claims 1 and 2, wherein amino carbonitriles of the formula
H₂N―(CH₂)ₘ―C≡N
where
m is 3, 4, 5 or 6 are employed.

4. A process as claimed in claim 3, wherein 6-aminocapronitrile is employed as amino carbonitrile.

5. A process as claimed in any of claims 1 to 4, wherein a 1-50% by weight solution of the amino carbonitrile in water or in water/org. solvent mixtures is employed.

## Revendications

1. Procédé de préparation de lactames cycliques par réaction de nitriles aminocarboxyliques avec de l'eau en présence de catalyseurs, **caractérisé en ce que** l'on entreprend la réaction en phase liquide en présence de catalyseurs hétérogènes à base de dioxyde de titane, d'oxyde de zirconium, d'oxyde de cérium et d'oxyde d'aluminium.

2. Procédé selon la revendication 1, **caractérisé en ce que** l'on entreprend la réaction à une température de l'ordre de 140 à 320°C.

3. Procédé selon l'une des revendications 1 à 2, **caractérisé en ce que** l'on met en oeuvre des nitriles aminocarboxyliques de formule
H₂N - (CH₂)ₘ - C ≡ N,
dans laquelle
m a une valeur de 3, 4, 5 ou 6.

4. Procédé selon la revendication 3, **caractérisé en ce que** l'on met en oeuvre en tant que nitrile aminocarboxylique du 6-amino-capronitrile.

5. Procédé selon l'une des revendications 1 à 4, **caractérisé en ce que** l'on met en oeuvre une solution à 1 à 50% en poids du nitrile aminocarboxylique dans de l'eau ou dans un mélange d'eau/solvant organique.
